# EUROPEAN PATENT SPECIFICATION

(11) **EP 1 379 146 B1**
(45) Date of publication and mention of the grant of the patent: **29.04.2009**
(21) Application number: 02707337.8
(22) Date of filing: 22.03.2002
(51) Int. Cl.: A23L 1/30, A61K 36/00

(54) **PREPARATION FOR THE PREVENTION AND/OR TREATMENT OF ALTERED BONE METABOLISM**
ZUSAMMENSETZUNG ZUR PRÄVENTION UND/ODER BEHANDLUNG VON VERÄNDERTEM KNOCHENMETABOLISMUS
PREPARATION DESTINEE A LA PREVENTION ET/OU AU TRAITEMENT DE METABOLISME OSSEUX MODIFIE

(30) Priority: 23.03.2001 EP 01201101
(43) Date of publication of application: 14.01.2004
(73) Proprietor: N.V. Nutricia, 2700 MA Zoetermeer (NL)
(72) Inventor: VAN HELVOORT, Adrianus, Lambertus, Bertholdus, NL-6703 ED Wageningen (NL); VAN NORREN, Klaske, NL-6871 LP Renkum (NL); GROS-VAN HEST, Marjan, NL-6871 TP Renkum (NL); LANSINK, Mirian, NL-3524 BJ Utrecht (NL)
(74) Representative: van Westenbrugge, Andries
(86) International application number: PCT/NL2002/000186
(87) International publication number: WO 2002/076241

(56) References cited:
- EP-A- 0 807 435
- WO-A-01/03687
- WO-A-91/11117
- WO-A-98/50026

## Description

The present invention relates to a preparation for the prevention and/or treatment of disorders related to altered bone metabolism.

Although it looks like an inert mineral substance, bone is a living, dynamic connective tissue that is constantly being renewed. It contains cells and specialized collagen fibers, encrusted with a crystalline mineral. Together, the minerals, cells, fibers and ground substance form the organic matrix or "osteoid". Bone mineral is a complex salt of calcium phosphate, citrate, boron, sodium, zinc, magnesium and fluorine. Most bones in the skeleton contain two types of bone tissue: compact bone and trabecular bone. Bone is constantly turned over by a combination of bone formation by osteoblasts and bone resorption by osteoclasts. If blood calcium levels are lower, resorption of the bone increases to fulfill calcium requirements elsewhere in the body.

Altered bone metabolism can be characterized by a misbalance between bone formation and bone resorption. It can occur in relation to several types of disorders. Examples are osteoporosis, osteopenia, Paget's disease, osteolytic metastasis in cancer patients, osteodistrophy in liver disease and the altered bone metabolism caused by renal failure or haemodialysis, bone fracture, bone surgery, pregnancy, anorexia nervosa and in immobile, malnourished and weight losing persons.

The major bone disease in the older population is osteoporosis. This disease is characterized by extensive bone loss leading to an increase in bone fragility and a greater liability for fractures. It causes considerable pain, disability, disfigurement and loss of independence, and is a cost and burden to health services. Internationally, more than 1.5 million fractures occur every year as a result of osteoporosis.

There are two types of osteoporosis recognized. The first typically occurs between the ages of 50 and 75 and affects six times as many women (post-menopausal osteoporosis) than men.

Bone loss is accelerated with aging. Most fractures occur in trabecular bone, especially the wrist and spinal vertebrae. The second type is referred to as senile osteoporosis and affects both men and women over 75 years of age and does not involve greater than normal bone loss. The risk factors for both types of osteoporosis are high caffeine intake, alcohol consumption, low body weight and low calcium intake.

The most prominent and well documented cause of post-menopausal osteoporosis is estrogen deficiency. After the menopause, the ovaries cease to produce this hormone, which directly relates to loss of bone mineral content. A very effective treatment for post-menopausal symptoms, including osteoporosis, is hormone replacement therapy (HRT), and this estrogen replacement effectively prevents the development of osteoporosis. Because HRT may have serious side effects, e.g. breast tissue growth stimulation, nutritional supplements for the prevention of osteoporosis are gaining popularity.

For other types of osteoporosis, the main cause has not been defined yet. Since elderly people usually have low amounts of calcium in their diet, their body calcium levels are low. The body, however, needs a stable blood calcium level for several other important functions, such as cell signaling, and thus bone resorption is increased to maintain adequate levels of this mineral in case of insufficient calcium intake.

The common approach to reduce bone loss with nutritional supplements is the use of phytoestrogens. These compounds are derived from botanical sources, such as soy or black cohosh, and have a molecular structure that resembles estrogen. The phytoestrogens can be classified as isoflavons (genistein, daidzein), lignans and coumestans. The mechanism of action of phytoestrogens has not been resolved yet. It is possible that their activity is related to the fact that they bind to the estrogen receptors in a similar way as estrogen, leading to comparable effects as conventional HRT. It is also possible that they directly act on osteoclasts and osteoblasts via an estrogen receptor independent mechanism.

WO-A-01/03687 teaches a method of inhibiting or reducing biosynthesis or bioactivity of endogenous steroid sex hormones in humans by administration of a combination of phytosterol(s) and phytoestrogen(s). The phytoestrogen is selected from the lignans, the isoflavones, the flavones and the coumestan classes. The use of such combinations is asserted to provide the benefits of lowered risk with respect to various cancers, while simultaneously preserving the benefits to cardiovascular and skeletal health.

WO-A-91/11117 discloses dietary supplements comprising vitamins and minerals together with other beneficial components among which bioflavonoids, and which are cardio- and cancer-protective as well as immunostimulatory.

WO-A-98/50026 discloses a composition for the treatment of menopausal symptoms and osteoporosis by means of a member of a small sub-group of isoflavones (comprising daidzein, genistein, biochanin and formononetin) that distinguishes by its ability to bind to estrogen receptors on animal (including human cells.

From EP-A-807.435 it is known to use luteolin as HMG CoA reductase indirect inhibitor for the treatment of atherosclerosis, heart infarct and other cardiovascular disorders. Luteolin helps by regulating the blood-cholesterol level.

Phytoestrogens and in particular isoflavones are for instance described in Alekel, D.L. et al, 2000, "Isoflavone-rich soy protein isolate attenuates bone loss in the lumbar spine of perimenopausal women", Am J Clin Nutr. 72:844-52; Messina, M. and Messina V., 2000, "Soyfoods, soybean isoflavones, and bone health: a brief overview", J Ren Nutr. 10:63-8; Erdman J.W. et al, 2000, "Provacative relation between soy and bone maintenance.", Am J Clin Nutr. 2000; 72:679-680.

In WO 98/50054 it is described that vegetable extracts can be used for the treatment of diseases or conditions characterized by increased bone resorption. Examples described are allium (onion, garlic), petroselinum (parsley), brassica (broccoli) and eruca (arrugula or roquette) extracts. In WO 00/20014 extracts of lycopersicon (tomato) are described. In WO 00/20015 extracts of anethum (dill) are mentioned.

It has now been found that a specific group of natural components can be used for the treatment and/or prevention of the above mentioned disorders related to altered bone metabolism. The present invention thus provides the use of apigenin and/or luteolin, combined with calcium, vitamin D and/or vitamin K, for the manufacture of a preparation for the treatment and/or prevention of a disorder related to altered bone metabolism.

The components of the invention (apigenin, luteolin) are preferably obtained from natural sources. Also mixtures thereof can be used. Further suitable are functional analogues such as glycosylated forms and plant extracts containing these components. The components according to the invention are used in an amount of 0.1 mg to 1 g, preferably 5 to 700 mg, more preferably 25 to 500 mg per daily dose.

The preparation for the treatment and/or prevention of a disorder related to altered bone metabolism according to the present invention can be a pharmaceutical preparation, such as a tablet, a capsule, a sachet or a suppository or a nutritional preparation, such as a nutritional supplement, a tube feeding, a complete food or a health promoting preparation. The preparation is suitable for the treatment of these disorders in mammals including humans.

The preparations preferably also contain one or more of the following:
a. calcium;
b. bone minerals, such as magnesium and boron;
c. gamma linolenic acid;
d. vitamins such as vitamin D and vitamin K;
e. estrogens or one or more estrogen mimicking compounds used in Estrogen Replacement Therapy;
f. biphosphonates and
g. isoflavones.

Calcium is added to increase calcium deposition in bones. The calcium source can be any suitable inorganic or organic compound containing calcium. Examples are inorganic calcium salts, for example calcium chloride, calcium phosphate, calcium sulphate, calcium oxide, calcium hydroxide or calcium carbonate. Examples of organic calcium compounds are milk powder or calcium caseinate, calcium citrate, calcium malate, calcium citrate malate or calcium lactate. The amount of calcium is preferably 200 to 1500 mg per daily dose.

Bone minerals are added to ensure strong bones. Preferably the preparation contains 100 mg to 500 mg magnesium and 2 mg to 6 mg boron per daily dose.

Gamma linolenic acid is used to regulate calcium metabolism, preferably in an amount of 25 mg to 100 mg per daily dose.

Vitamins are added as cofactors for optimal bone metabolism. Preferably, daily vitamin K dose should be 25 µg to 5 mg vitamin K. Vitamin D is used to increase calcium uptake from the gut. Preferably 5 µg to 20 µg (200 IU to 800 IU) per daily dose is present in the preparation.

Estrogens or one or more estrogen mimicking compounds used in Estrogen Replacement can also be included in the preparation for the treatment and/or prevention of osteoporosis. Examples of these compounds are phytoestrogens, like genistein, lignans or coumerans or pharmaceutical preparations like 17β-estradiol, esterified estrogens, estrone sulfate, conjugated equine estrogen, and ethinylestradiol. For the phytoestrogens the amount of these compounds is 5-100 mg. For the pharmaceutical preparations the active amount is defined by the instructions of the manufactures.

One or more biphosphonates are used to inhibit the osteoclastic bone resorption. Examples of these compounds are alendronate and risedronate. Preferably the amount of these compounds is 5 mg to 50 mg.

Isoflavones can be obtained (isolated) from soy or black cohosh or can be synthetic isoflavones. Isoflavones can be added in an amount of 10 to 75 mg per daily dose.

The preparations of the invention can further contain other sources of energy, such as fats and carbohydrates, proteins, vitamins, minerals, fibers, flavors, preservatives, colorants, sweeteners, etc.

The active components according to the invention can be used as pure substances but can also be in the form of extracts of plants or parts thereof. Morin can for example be used in the form of extracts of jackfruit (*Artocarpus heterophyllus*) and white mulberry (*Morus alba*). Luteolin can for example be used in the form of extracts of peanut shells (*Arachis hypogaea*), artichock (*Cynara scolymus*) and celery (*Apium graveolens*). Apigenin can for example be used in the form of extracts of chamomillae (*Matricaria recutita*) or celery (*Apium graveolens*).

Extracts can be used by methods known in the art, e.g. by extracting the starting material with water or a food grade solvent, or mixtures thereof. A preferred food grade solvent is ethanol. After extraction the liquid phase containing the active ingredients can be concentrated or dried by evaporation or freeze drying.

Extracts of petroselinum (parsley), eruca (arrugula or roquette), lycopersicon (tomato) and anethum (dill) are excluded from the invention.

The components and preparations of the present invention can in particular be used for disorders characterized by altered bone metabolism. The disorder can be osteoporosis, osteopenia, Paget's disease, osteolytic metastasis in cancer patients, osteodistrophy in liver disease or the altered bone metabolism caused by renal failure or haemodialysis, bone fracture, bone surgery, pregnancy, anorexia nervosa, and in immobile, malnourished and weight losing persons.

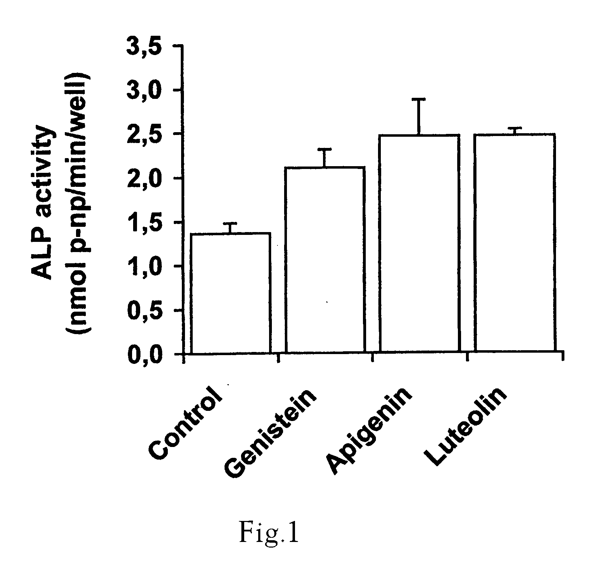

The invention will now be illustrated by means of the following examples and the attached figure 1 which shows the alkaline phosphatase activity in mouse bone marrow cells after 3 weeks exposure to the components of the invention.

### Examples

### 1. Formulations

### Nutritional supplement 1

| **Components** | **Amount per serving** | |
|---|---|---|
| Apigenin | 15 | mg |
| Luteolin | 15 | mg |

### Nutritional supplement 2:

| **Components** | **Amount per serving** | |
|---|---|---|
| Morin | 10 | mg |
| Apigenin | 20 | mg |
| Luteolin | 5 | mg |

### Nutritional supplement 3:

| **Components** | **Amount per serving** | |
|---|---|---|
| Calcium | 800 | mg |
| White Mulberry extract (providing glycosylated morin) | 10 | mg |
| Chamomilla extract (providing glycosylated apigenin) | 10 | mg |
| Artischock extract (providing glycosylated luteolin). | 10 | mg |
| *Amounts of active components are expressed as aglycons* | | |

### Nutritional supplement 4:

| **Components** | **Amount per serving** | |
|---|---|---|
| Calcium | 800 | mg |
| Vitamin D3 | 200 | IU |
| Morin | 10 | mg |
| Apigenin | 10 | mg |
| Luteolin | 100 | mg |

### Nutritional supplement 5:

| **Components** | **Amount per serving** | |
|---|---|---|
| Calcium as Calcimate® | 800 | mg |
| Vitamin D3 | 200 | IU |
| Magnesium | 200 | mg |
| Boron | 2 | mg |
| Morin | 5 | mg |
| Apigenin | 25 | mg |
| Luteolin | 20 | mg |
| Gamma linolenic acid | 25 | mg |

### Nutritional supplement 6:

| **Components** | **Amount per serving** | |
|---|---|---|
| Calcium as Calcimate® | 800 | mg |
| Vitamin D3 | 200 | IU |
| Magnesium | 200 | mg |
| Boron | 2 | mg |
| Isoflavones | 30 | mg |
| Apigenin | 50 | mg |
| Gamma linolenic acid | 25 | mg |
| Vitamin K1 | 500 | µg |

### Nutritional supplement 7:

| **Components** | **Amount per serving** | |
|---|---|---|
| Calcium | 800 | mg |
| Vitamin D3 | 200 | IU |
| Black cohosh providing isoflavones | 40 | mg |
| Soy extract providing glycosylated isoflavones | 30 | mg |
| Morin | 10 | mg |
| Apigenin-7-glucoside | 10 | mg |
| Luteolin-4' glucoside | 10 | mg |
| *Amounts of active components and isoflavones are expressed as aglycons* | | |

### Stimulating effect on mouse bone marrow cells

The following experiment is illustrated in Figure 1, which shows the alkaline phosphatase activity in mouse bone marrow cells after 3 weeks exposure to the components of the invention.

### Experiment

Bone marrow cells were isolated from six months old female NMRI mice. After washing, the cells were diluted in phenol red free α-MEM (1% p/s, 15% charcoal treated FCS) and seeded in 24-well plates (1.25* 10⁶ cells/well). After 24 hours incubation (37°C, 5% CO₂) the cells were exposed to the active components (1*10⁻⁵, 1*10⁻⁶ and 1*10⁻⁷ M) together with ascorbic acid (50 µg/ml) and β-glycerolphosphate (1 mM). Exposure media were refreshed every 3 days.

After 2 and 3 weeks of incubation, cells were washed with PBS and stored at -20°C. After lysis DNA amounts and alkaline phosphatase activities were determined as described below. The DNA amounts are measured to determine to the number of cells and the alkaline phosphatase activity is used as a marker for osteoblast differentiation.

### Lysis

300 µl lysis buffer (1 x SSC/0.01% SDS) was added to the frozen cells. After 1 hour incubation (37°C) the plates were sonified for 10 minutes and cell suspensions were used for ALP activity measurements.

### Alkaline phosphatase (ALP) activity assay

25 µl cell lysate was transferred in duplicate to 96-well plates. 25 µl of 1*SSC/0.01%SDS served as a negative control. 175 µl of ALP-assay buffer (10 mM glycine, 0.1 mM MgCl₂, 0.01 mM ZnCl₂, 6 mM p-nitrophenylphosphate, pH 10.5), was added to every well. ALP activity was measured at a wavelength of 405 nm at room temperature during 10 minutes at 30-second intervals. The results of this assay are shown in figure 1, which shows the alkaline phosphatase activity in mouse bone marrow cells after 3 weeks exposure to the active components. Activity is expressed as nmol p-nitrophenol production/minute/well. (Mean ± sem, 2 independent experiments measured in duplicate)

Figure 1 shows that incubations of mice bone marrow cells with 1 mM apigenin and luteolin result in an increased alkaline phosphatase signal in differentiated osteoblasts. The stimulation is more pronounced than the effect of the isoflavone genistein of which bone loss prevention has been shown *in vivo* (rat and human). Similar results were obtained with morin (data not shown).

## Claims

1. Use of apigenin and/or luteolin, combined with calcium, vitamin D and/or vitamin K, for the manufacture of a preparation for the treatment and/or prevention of a disorder related to altered bone metabolism in a mammal.

2. Use according to claim 1, comprising the use of luteolin.

3. Use according to claim 1 or 2, wherein the disorder is osteoporosis, osteopenia, Paget's disease, osteolytic metastasis in cancer patients, osteodistrophy in liver disease or the altered bone metabolism caused by renal failure or haemodialysis, bone fracture, bone surgery, pregnancy, anorexia nervosa, and in immobile, malnourished and weight-losing patients.

4. Use according to any one of the preceding claims, wherein the preparation comprises one or more extracts selected from the group consisting of peanut shells (*Arachis hypogaea*), artichoke (*Cynara scolymus*), celery (*Apium graveolens*) and chamomile (*Matricaria recutita*).

5. Use according to any of the preceding claims, wherein the preparation is a pharmaceutical preparation, such as a tablet, a capsule, a sachet or a suppository, or a nutritional preparation, such as a nutritional supplement, a tube feeding, a complete food or a health promoting preparation.

6. Use according to any of the preceding claims, in combination with isoflavones.

7. Preparation containing
a. apigenin and luteolin, and
b. an ingredient providing an effective amount of calcium, vitamin D and/or vitamin K.

8. Preparation according to claim 7, wherein the preparation further comprises an effective amount of isoflavones.

9. Preparation according to claim 8, wherein the isoflavones are isolated from soy or black cohosh.

10. Preparation according to any of claims 7 to 9, which comprises extracts selected from the group consisting of peanut shells (*Arachis hypogaea*), artichock (*Cynara scolymus*)*,* celery (*Apium graveolens*) and chamomillae (*Matricaria recutita*).

11. Preparation according to any of claims 7 to 10, further comprising one or more members selected from the group consisting of magnesium, boron and gamma linolenic acid.

12. Preparation according to any of claims 7 to 11, further comprising estrogens or one or more estrogen mimicking compounds used in Estrogen Replacement Therapy.

13. Preparation according to any of claims 7 to 12, further comprising one or more biphosphonates.

14. Use of a preparation according to any of claims 7 to 13 for the manufacture of a preparation for treatment and/or prevention of a disorder related to altered bone metabolism.

15. Use according to claim 14, wherein the disorder is osteoporosis, osteopenia, Paget's disease, osteolytic metastasis in cancer patients, osteodistrophy in liver disease or the altered bone metabolism caused by renal failure or haemodialysis, bone fracture, bone surgery, pregnancy, anorexia nervosa, and in immobile, malnourished and weight-losing patients.

## Patentansprüche

1. Verwendung von Apigenin und/oder Luteolin kombiniert mit Kalzium, Vitamin D und/oder Vitamin K zur Herstellung eines Präparates für die Behandlung und/oder Prävention einer Erkrankung in Beziehung zu veränderten Knochenmetabolismus in einem Säugetier.

2. Verwendung nach Anspruch 1, umfassend die Verwendung von Luteolin.

3. Verwendung nach Anspruch 1 oder 2, wobei es sich bei der Erkrankung um Osteoporose, Osteopenia, Paget-Erkrankung, Osteolytische Metastase in Krebskranken, Osteodystrophie in Leberkrankheit oder der veränderte Knochenmetabolismus hervorgerufen durch Niereversagen oder Hämodialyse, Knochenbruch, Knochenoperation, Schwangerschaft, Anorexia Nervosa, und in immobile, unterernährte und abnehmende Patienten handelt.

4. Verwendung nach einem der vorhergehenden Ansprüche, wobei das Präparat ein oder mehr Extrakten ausgewählt aus der Gruppe bestehend aus Erdnussschale (*Arachis Hypogaea*)*,* Artischocke (*Cynara scolymus*)*,* Sellerie (*Apium graveolens*) und Kamille (*Matricaria recutita*) umfasst.

5. Verwendung nach einem der vorhergehenden Ansprüche, wobei es sich bei dem Präparat um ein pharmazeutisches Präparat, wie eine Tablette, eine Kapsel, ein Sachet oder ein Zäpfchen, oder ein Nährpräparat, wie ein Nahrungssupplement, eine Sondenernährung, eine Totalernährung oder ein gesundheitsförderndes Präparat.

6. Verwendung nach einem der vorhergehenden Ansprüche, in Kombination mit Isoflavonen.

7. Präparat umfassend
a. Apigenin und Luteolin, und
b. ein Ingrediens verschaffende eine wirksame Menge von Kalzium, Vitamin D und/oder Vitamin K.

8. Präparat nach Anspruch 7, wobei das Präparat weiterhin eine wirksame Menge von Isoflavonen umfasst.

9. Präparat nach Anspruch 8, wobei die Isoflavonen isoliert sind aus Soja oder Traubensilberkerze.

10. Präparat nach einem der Ansprüche 7-9, das Extrakten ausgewählt aus der Gruppe bestehend aus Erdnussschale (*Arachis Hypogaea*)*,* Artischocke (*Cynara scolymus*)*,* Sellerie (*Apium graveolens*) und Kamille (*Matricaria recutita*) umfasst.

11. Präparat nach einem der Ansprüche 7-10, weiterhin umfassend ein oder mehr Teile ausgewählt aus der Gruppe bestehend aus Magnesium, Bor und Gammalinolensäure.

12. Präparat nach einem der Ansprüche 7-11, weiterhin umfassend Östrogen oder ein oder mehr Östrogenimitierende Verbindungen verwendet in Östrogenersatztherapie.

13. Präparat nach einem der Ansprüche 7-12, weiterhin umfassend ein oder mehr Biophosphonaten.

14. Verwendung eines Präparates nach einem der Ansprüche 7-13 zur Herstellung eines Präparates für die Behandlung und/oder Prävention einer Erkrankung in Beziehung zu veränderten Knochenmetabolismus.

15. Verwendung nach Anspruch 14, wobei es sich bei der Erkrankung um Osteoporose, Osteopenia, Paget-Erkrankung, Osteolytische Metastase in Krebskranken, Osteodystrophie in Leberkrankheit oder der veränderte Knochenmetabolismus hervorgerufen durch Niereversagen oder Hämodialyse, Knochenbruch, Knochenoperation, Schwangerschaft, Anorexia Nervosa, und in immobile, unterernährte und abnehmende Patienten handelt.

## Revendications

1. Utilisation d'apigénine et/ou de lutéoline combinée avec du calcium, de la vitamine D et/ou de la vitamine K pour la manufacture d'une préparation pour le traitement et/ou la prévention d'un désordre lié à l'altération du métabolisme des os chez un mammifère.

2. Utilisation selon la revendication 1, comprenant l'utilisation de la lutéoline.

3. Utilisation selon la revendication 1 ou 2, où le désordre est l'ostéoporose, l'ostéopénie, la maladie de Paget, la métastase ostéolytique chez des patients atteints de cancer, l'ostéodystrophie dans les maladies du foie ou le métabolisme des os a été altéré à cause d'une défaillance rénale ou d'hémodialyse, la fracture osseuse, la chirurgie osseuse, la grossesse, l'anorexie nerveuse, et chez les patients immobiles, malnourrits et perdant du poids.

4. Utilisation selon l'une quelconque des revendications précédentes, où la préparation comprend un ou plusieurs extraits sélectionnés parmi le groupe consistant en des enveloppes de cacahuètes (*Arachis hypogaea*)*,* de l'artichaut (*Cynara scolymus*)*,* du céleri (*Apium graveolens*) et de la camomille (*Matricaria recutita*).

5. Utilisation selon l'une quelconque des revendications précédentes, où la préparation est une préparation pharmaceutique, telle qu'une tablette, une capsule, un sachet ou un suppositoire, ou une préparation nutritionelle telle qu'un supplément nutritionel, une préparation pour sonde, une nourriture complète ou une préparation améliorant la santé.

6. Utilisation selon l'une quelconque des revendications précédentes, en combinaison avec des isoflavones.

7. Préparation comprenant
a. de l'apigénine et de la lutéoline, et
b. un ingrédient représentant une quantité effective de calcium, vitamine D et/ou de vitamine K.

8. Préparation selon la revendication 7, où la préparation comprend en outre une quantité effective d'isoflavones.

9. Préparation selon la revendication 8, où les isoflavones sont isolées du soja ou du cohosh noir.

10. Préparation selon l'une quelconque des revendications 7-9, qui comprend des extraits sélectionnés parmi le group qui consiste en des enveloppes de cacahuètes (*Arachis hypogaea*)*,* de l'artichaut (*Cynara scolymus*)*,* du céleri (*Apium graveolens*) et de la camomille (*Matricaria recutita*).

11. Préparation selon l'une quelconque des revendications 7-10, qui comprend en outre un ou plusieurs membres sélectionnés parmi le groupe qui consiste en du magnésium, du bore et de l'acide gamma linolénique.

12. Préparation selon l'une quelconque des revendications 7-11, qui comprend en outre des estrogènes ou un ou plusieurs composants imitant les estrogènes et qui sont utilisés en Thérapie de Remplacement de l'Estrogène.

13. Préparation selon l'une quelconque des revendications 7-12, qui comprend en outre un ou plusieurs biphosphonates.

14. Utilisation d'une préparation selon l'une quelconque des revendications 7-13 pour la manufacture d'une préparation pour le traitement et/ou la prévention d'un désordre lié à l'altération du métabolisme des os.

15. Utilisation selon la revendication 14, où le désordre est l'ostéoporose, l'ostéopénie, la maladie de Paget, la métastase ostéolytique chez des patients atteints de cancer, l'ostéodystrophie dans les maladies du foie ou le métabolisme des os a été altéré à cause d'une défaillance rénale ou d'hémodialyse, la fracture osseuse, la chirurgie osseuse, la grossesse, l'anorexie nerveuse, et chez les patients immobiles, malnourrits et perdant du poids.
